(19) Europäisches Patentamt
European Patent Office
Office européen des brevets

(11) EP 0 470 000 B1

(12) # FASCICULE DE BREVET EUROPEEN

(45) Date de publication et mention de la délivrance du brevet:
**12.11.1997 Bulletin 1997/46**

(51) Int Cl.[6]: **C07C 209/18**, C07C 211/52, C07C 231/08, C07C 41/14, C07C 37/16

(21) Numéro de dépôt: **91402158.9**

(22) Date de dépôt: **01.08.1991**

(54) **Réactif d'allylation et un procédé de synthèse utilisant ce réactif**

Allylierungsreagens und ein es verwendendes Herstellungsverfahren

Allylation reagent and method of synthesis using it

(84) Etats contractants désignés:
**AT BE CH DE DK ES FR GB GR IT LI LU NL SE**

(30) Priorité: **02.08.1990 FR 9009887**
**02.08.1990 FR 9009886**
**02.08.1990 FR 9009888**
**02.08.1990 FR 9009889**

(43) Date de publication de la demande:
**05.02.1992 Bulletin 1992/06**

(73) Titulaire: **RHONE-POULENC CHIMIE**
**92408 Courbevoie Cédex (FR)**

(72) Inventeurs:
- **Grosselin, Jean-Michel**
**F-69340 Francheville (FR)**
- **Kempf, Hubert**
**F-68200 Mulhouse (FR)**
- **Lecouve, Jean-Pierre**
**F-68100 Mulhouse (FR)**

(74) Mandataire: **Ricalens, François et al**
**RHONE-POULENC CHIMIE,**
**Direction de la Propriéte Industrielle,**
**25, quai Paul Doumer**
**92408 Courbevoie Cédex (FR)**

(56) Documents cités:

| | |
|---|---|
| **EP-A- 0 205 391** | **EP-A- 0 385 835** |
| **EP-A- 0 407 256** | **FR-A- 2 305 434** |

- **TETRAHEDRON LETTERS. no. 43, Septembre 1970, OXFORD GB pages 3821 - 3824; K. E. ATKINS ET AL: 'PALLADIUM CATALYZED TRANSFER OF ALLYLIC GROUPS'**
- **RESEARCH DISCLOSURE. vol. 169, Mai 1978, HAVANT GB page 35; 'PREPARATION OF ALLYL AMINES'**
- **JOURNAL OF ORGANIC CHEMISTRY. vol. 50, no. 9, 3 Mai 1985, EASTON US pages 1523 - 1529; J. TSUJI ET AL: 'ALLYLIC CARBONATES. EFFICIENT ALLYLATING AGENTS OF CARBONUCLEOPHILES IN PALLADIUM-CATALYZED REACTIONS UNDER NEUTRAL CONDITIONS'**
- **BULLETIN OF THE ACADEMY OF SCIENCES OF THE USSR DIVISION OF CHEMICAL SCIENCE vol. 34, no. 11, 20 Mai 1986, NEW YORK US pages 2374 - 2378; U. M. DZHEMILEV ET AL: 'CYCLOPENTADIENYL DERIVATIVES OF MAGNESIUM AND SODIUM IN CROSS-COMBINATION REACTION WITH ALLYL COMPOUNDS CATALYZED BY Pd COMPLEXES'**
- **CHEMISTRY LETTERS. no. 3, Mars 1986, TOKYO JP pages 293 - 294; Y. TSUJI ET AL: 'PLATINUM COMPLEX CATALYZED TRANSFORMATION OF AMINE. N-ALKYLATION AND N-ALLYLATION USING PRIMARY ALCOHOLS'**
- **CHEMICAL ABSTRACTS, vol. 110, no. 15, 10 Avril 1989, Columbus, Ohio, US; abstract no. 134848F, S. A. LEBEDEV ET AL: 'ALLYLATION OF ACETANILIDES BY ALLYL ACETATE UNDER CONDITIONS OF COMBINED METAL-COMPLEX AND PHASE-TRANSFER CATALYSIS'**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 470 000 B1

## Description

La présente invention concerne un procédé de synthèse de composés allylés. Elle concerne plus particulièrement l'allylation d'anilines telles que les métatrifluorométhylanilines.

Les N-allylations d'amides et notamment d'anilides, d'anilines et de phénol, sont souvent difficiles et nécessitent l'utilisation de réactifs qui, tels les halogènures d'alcoyles sont coûteux et engendrent des quantités importantes de rejets salins.

Par ailleurs, la N-allylation d'anilides peut être une étape importante dans la synthèse de nombreux composés ; la réaction peut conduire à des mono- ou polyallylations et il peut être capital d'obtenir un type d'allylation qui soit sélectif vis-à-vis des autres. Ainsi lors de la synthèse du N-allyl, N-dichloroacétylmétatrifluorométhylanilide, intermédiaire de synthèse d'un herbicide important, une étape d'allylation de la métatrifluorométhylaniline (m-TFMA) doit être une monoallylation aussi sélective que possible vis-à-vis de la diallylation.

C'est pourquoi un des buts de la présente invention est de fournir un procédé qui soit capable d'allyler des anilides, même lorsqu'ils sont désactivés par des groupes électro-attracteurs.

C'est pourquoi un autre but de la présente invention est de fournir un procédé qui soit capable d'allyler des anilines en utilisant des réactifs peu chers et faciles d'accès.

C'est pourquoi un des buts de la présente invention est de fournir un procédé qui soit capable d'allyler des anilines même désactivées par des groupes électro-attracteurs.

C'est pourquoi un des buts de la présente invention est de fournir un procédé qui soit capable d'allyler des phénols même désactivés par des groupes électro-attracteurs.

C'est pourquoi un des buts de la présente invention est de fournir un procédé qui soit capable d'allyler tout substrat nucléophile, notamment non ionique (comme les organometalliques), même aussi mauvais que les amides.

C'est pourquoi un autre but de la présente invention est de fournir un réactif d'allylation utilisable pour le procédé ci-dessus.

Ces buts et d'autres qui apparaîtront par la suite sont atteints
au moyen d'un procédé mettant en oeuvre un réactif allylant comportant au moins :

- une phase aqueuse
- un dérivé allylique de préférence éther ou alcool allylique, et
- un catalyseur comportant au moins un élément de la colonne VIII de la classification périodique des éléments(cf. Bull. Soc. Chim. 1966 N°1 supplément).

L'utilisation de dérivés des alcools allyliques avaient déjà été décrit, mais jamais en utilisant un réactif comme décrit ci-dessus. Le document **Tétrahedron Letters n° 43, page septembre 1970, 3821 à 3824** décrit une technique d'allylation au moyen d'alcool en présence de palladium divalent et de triphénylphosphine en milieu organique. Le palladium est introduit sous forme d'acétylacétonate, cependant que de l'acétylacétone est ajouté, en outre l'allylation est ainsi réalisée sur l'excellent nucléophile que constituent les ions acétylacétonates. La réaction est également réputée marcher sur lesdits alcoylamines.

Le document **Chemistry Letters p. 293-294, 1986** décrit des alcoylations sur des amines et sur des anilines en présence de platine et de chlorure d'étain. Les réactions donnent des résultats relativement médiocres et aucune aniline désactivée n'est décrite.

Le document **Chemical Abstract Volume 110 résumé 134848F** décrit l'allylation de l'anion de l'acétanilide à partir d'acétate d'allyle dans des conditions combinées de complexations métalliques et de catalyse au moyen de transfert de phase.

Les conditions, sont extrêmement basiques avec le risque d'hydrolyse si l'allylation était réalisée en phase aqueuse.

Le document **J. Org. Chem. 1985, 50, 1523-1529** décrit un système d'allylation de carbanion, mais ne décrit pas l'allylation des anilines désactivées et des phénols.

Le document **Research Disclosure**, **Mai 1978, 169** décrit la synthèse de diéthyl allylamine dans divers solvants, y compris dans l'eau, en utilisant les acétates d'allyle et les éthers d'allyle. Elle ne décrit ni l'utilisation d'agents de coordination hydrosolubles ni l'utilisation de cette technique pour des amines désactivées.
Aucun de ces documents n'a montré l'avantage d'opérer avec le catalyseur en phase aqueuse.

Enfin la demande de brevet européen EP-A-0 407 256 publiée postérieurement au dépôt de la présente demande, décrit une technique utilisant une phase liquide mais ne décrit précisément la réaction qu'avec des esters allyliques et en association avec des nitriles.

- le dérivé allylique est, de préférence, de formule (III) suivante :

$$Z\text{-}O\text{-}C(R_1)(R_2)\text{-}C(R_3)\text{==}C(R_4)(R_5)$$

où

- . $R_1$ représente un hydrogène ou un radical alcoyle, de préférence à 1 ou 2 atomes de carbone ;
- . $R_2$ représente un hydrogène ou un radical alcoyle, de préférence à 1 ou 2 atomes de carbone ;
- . $R_3$ représente un hydrogène ou un radical alcoyle ;
- . $R_4$ représente un hydrogène ou un radical alcoyle ;
- . $R_5$ représente un hydrogène ou un radical alcoyle, et Z représente un hydrogène ou un reste déduit d'un alcool par enlèvement d'un groupe hydroxyle ;

le nombre total d'atomes de carbone du dérivé allylique étant avantageusement au plus égal à 50 de préférence à 30 (ici le terme alcool doit être compris "lato sensu" et englobe les phénols) par enlèvement d'un groupe hydroxyle.

- un catalyseur comportant au moins un élément de la colonne VIII de la classification périodique des éléments.

L'éther peut être fait "in situ" à partir d'alcool(s) ; dans ces cas là, il est préférable que la teneur stationnaire en éther soit telle que le rapport éther-alcool soit supérieur à 1/2 %, avantageusement supérieure à 5 %, de préférence compris entre 1/10e et 1/5e.

Le terme alcoyle est pris avec la signification du dictionnaire de Chimie Duval.

Il est préférable qu'au moins 2, avantageusement 3, de préférence 4 des radicaux $R_1$ à $R_5$ soient au plus de deux carbones; toutefois, au moins un des radicaux $R_1$ à $R_5$ peut être tel que l'alcool allylique soit un alcool lourd, par exemple de série aromatique, de type terpénique ou de série stéroïdique.

Ainsi, au moins 1 radical et au plus 3 radicaux $R_1$ à $R_5$ peuvent être des radicaux aryles, polycycliques, condensés ou non, homo ou hétérocycliques.

Le dit réactif peut dans le procédé selon la présente invention, être mis en contact avec tout substrat nucléophile, notamment non anionique (anionique comme le sont par exemple les espèces réputées actives dans les organométalliques en particulier dans le cas des synthèses par carbanions, les espèces rendues nucléophiles par arrachement d'hydrogène mobile pour former un carbanion sont réputés anioniques ), y compris les phénols et les anilines désactivées par au moins un substituant électro-attracteur sur, ou par la présence d'un hétéroatome dans le noyau aromatique.

La réaction donne également de très bons résultats lorsque le noyau aromatique est désactivé par la présence de groupe(s) attracteur(s).

Par radical aryle désactivé, il convient de comprendre appauvri en électrons et avec une densité électronique au plus égale à celle du benzène, voire au plus voisine de celle d'un halobenzène. Cet appauvrissement peut être dû à la présence d'un hétéroatome dans le cycle aromatique lorsque il a six chaînons, comme par exemple à titre d'enseignement dans la pyridine, la quinoléine.

Bien évidemment, l'appauvrissement électronique peut être provoqué aussi par des groupes électro-attracteurs ; la pauvreté en électrons peut être due à ces deux causes.

Ainsi, ledit aryle désactivé porte en général au moins un substituant de préférence choisi parmi les groupes attracteurs par effet donneur ou par effet mésomère tel que défini dans l'ouvrage de référence en Chimie Organique "Advanced Organic Chemistry" par M.J. MARCH, 3ème édition, Editeur WILLEY, 1985, cf. notamment p. 17 et 238. Comme exemple de groupe attracteur, on peut citer : $NO_2$, $CF_3$, CN, COX (X = Cl, Br, F,OR), CHO, Cl, Br.

Ainsi l'allylation donne des résultats particulièrement intéressants lorsque le procédé est appliqué à un composé de formule V) suivante :

$$(R)_n\ Ar\text{-}Y\text{-}H \qquad (V)$$

dans laquelle :

- Ar représente un radical aromatique mono ou polycyclique, conden-sé ou non, homo ou hétérocyclique,
- R représente au moins un substituant choisi indépendamment parmi le chlore, le brome, les radicaux alkyles linéaires, ramifiés, cycliques saturés ou insaturés éventuellement substitués, éther, alcoxy éventuellement substitué, aryle éventuellement substitué, aryloxy, amino, hydroxy, carboxylate, acyloxy, ester, amido, nitrile, acide,

- n est **égal à zéro ou est** un nombre entier égal à 1, 2, 3 ou 4,
- Y représente un atome de chalcogène, de préférence d'oxygène, ou un groupement NR' avec R' représentant un acyle léger (de 1 à 10, avantageusement de 1 à 6, de préférence de 2 à 5 atomes de carbone eventuellement polyhalogéné préférentiellement mono-, di-, ou trihalogéné), un alkyle léger (de 1 à 10, de préférence de 1 à 6 atomes de carbone) voire un radical aryle ou avantageusement un atome d'hydrogène.

Sont visées plus particulièrement les anilines désactivées de formule II suivante :

$$A\text{-}Ar\text{-}N\text{-}H_2 \qquad (II)$$

où :

- A représente un substituant tel que défini par R, de préférence un groupe attracteur, notamment perfluoroalkyle, ou un atome d'hydrogène ;
- Ar a la même signification que précédement et les phénols présentant la formule IV suivante :

$$(R)_n\ Ar\text{-}O\text{-}H \qquad (IV)$$

où :

- Ar,R et n ont la même signification que précédement.

Ainsi la réaction donne également de très bons résultats lorsque le noyau aromatique est désactivé par la présence de groupe(s) attracteur(s). Le nombre total de carbones des substrats dépasse rarement 50, bien qu'il n'yait aucune contre indication pour les substrats plus lourds.

Les conditions réactionnelles avantageuses pour la mise en oeuvre d'une telle réaction sont les suivantes :

La température est en général supérieure à l'ambiante et inférieure ou égale à la température de reflux. Lorsqu'il convient de travailler à d'autres températures, on utilise en général une température comprise entre 100°C et 200°C. La pression utilisée est avantageusement la pression d'équilibre du réactif à la température d'utilisation bien qu'une pression supérieure ne soit en aucun cas nuisible.

En outre, la réaction est de préférence menée en absence d'oxydant et notamment d'oxygène qui présente l'inconvénient d'oxyder les phosphines ou équivalents. C'est la raison pour laquelle les réactions sont menées sous atmosphère inerte (tels que gaz rare, azote...) sur des réactifs préalablement dégazés si les phosphines ou équivalents utilisés sont particulièrement fragiles.

L'allylation peut être notamment une N, O ou C allylation. Lorsque le radical allyle est palindrome, le sens de l'allylation importe peu. Sinon à titre indicatif, elle se fait essentiellement selon les règles connues de l'homme de métier en position 1 ou 3 du radical allylique, régles où l'encombrement joue un rôle important (allylation par le coté le moins encombré).

Lorsque l'éther est un éther phénolique, l'allylation prépondérante est une C allylation interne en position ortho. Ainsi, lorsque l'allylation est pratiquée sur un phénol (lato sensu), l'éther phénolique obtenu peut se transposer par une C allylation interne en position ortho, libérant la fonction phénol pour une deuxième allylation.

Les métaux donnant les meilleurs résultats catalytiques sont les métaux de la mine du platine ; toutefois il peut être économiquement intéressant d'utiliser des métaux moins lourds en raison de leur coût bien moindre ; au sein de la famille des métaux de la mine du platine, chacun d'entre eux présente des spécificités qui les rendent plus ou moins intéressants selon les cas.

A titre d'exemple, lors de l'allylation des anilides, en général le métal donnant le meilleur résultat est le palladium, en général activé par au moins une pnictine ; toutefois lorsque l'anilide dérive d'un acide alpha halogèné ou d'une manière générale lorsque le substrat présente un halogène activé, la catalyse au palladium peut conduire à de nombreux sous-produits. Dans ces cas là, il a été démontré que l'utilisation de platine, de préférence activé par au moins une pnictine (arsine, stibine, phosphine...) donne des résultats à la fois significatifs et sélectifs.

Dans le cas de l'allylation de composés bi- ou poly- substituables, il peut être souhaitable de ne pas trop augmenter la cinétique de la réaction, notamment lorsque l'on désire ne réaliser qu'une mono- ou oligoallylation. Dans ces situations, il peut même être opportun d'ajouter des agents ralentissant la réaction tels que le trioxyde d'antimoine ou de bismuth.

Lorsque la sélectivité entre oligo- et polyallylation n'est pas jugée suffisante, on peut pour l'améliorer, jouer sur

l'excès d'éther allylique, ou dérivé, par rapport au substrat, par exemple en limitant l'excès stoechiométrique par rapport à la réaction désirée à 1/2, avantageusement à 1/4, de préférence à 1/10 ou bien en travaillant avec un excès de substrat.

Ainsi la présente invention enrichit l'état de la technique d'une palette de réactifs aux nuances variées utilisables et adaptables à de nombreux cas de figures.

Les caractéristiques catalytiques des éléments de la colonne VIII peuvent être modifiées par des agents de coordination, choisis en général parmi les dérivés organiques des éléments de la colonne V connus pour avoir cette capacité à coordiner tels que les phosphines, arsines, les stibines ou les nitriles, notamment aromatiques tels que le benzonitrile les composés organooxygènés de ces éléments de la colonne V ,comme par exemple les esters de l'acide phosphoreux, les phosphonates et phosphinates. Les éléments de la colonne V préférés sont ceux dont la période est d'un rang plus élevé que celui de celle de l'azote.

Lesdits agents de coordination sont avantageusement des dérivés hydrocarbonés des éléments de la colonne V . Lesdits dérivés hydrocarbonés des éléments de la colonne V dérivent de l'azote telles les amines, du phosphore telles les phosphines, de l'arsenic, telles les arsines et de l'antimoine telles les stibines ; ces composés sont, par analogie avec le terme de pnicture, désignés dans la présente description sous le terme de pnictines. En d'autre termes une pnictine est au pnicture ce qu'une phosphine est au phosphure ; ainsi sous ce vocable convient-il de comprendre le pnicture d'hydrogène et les composés derivant du pnicture d'hydrogène par remplacement de l'un, de deux ou des trois hydrogènes du pnicture d'hydrogène par une chaine, ou un radical, hydrocarbonnée.

Ils sont avantageusement choisis parmi les dérivés hydrocarbonés du phosphore tels que les phosphines.

Avantageusement, ledit catalyseur comporte comme pnictine, une trialcoylphosphine, de préférence une triphénylphosphine. Ladite phosphine et ledit métal de la colonne VIII sont avantageusement sous forme de tetrakis phosphine métal.

Compte tenu de la paresse à réagir de certains substrats, faiblement nucléophiles, on peut pour accélérer l'allylation, envisager d'ajouter de faibles quantités de sel stanneux, avantageusement au plus égales à celles du métal de la colonne VIII, de préférence environ dix fois moins en mole de sels stanneux tel que $SnCl_2$.

Il convient de souligner que l'un des buts importants de la présente invention est fournir un réactif qui permette l'allylation désirée en particulier une monoallylation lorsque cela est voulu. La présence de sels stanneux favorise fortement la diallylation des anilines.

C'est pourquoi, il est en général préférable que le catalyseur présente une teneur en étain aussi faible que possible. Avantageusement, le rapport molaire entre l'étain et l'élément de la colonne VIII de la classification périodique des éléments est inférieur à $10^{-2}$, de préférence à $10^{-4}$.

Dans le cas plus particulièrement visé par l'invention où l'on désire réaliser une N-monoallylation sur une aniline désactivée par au moins un substituant électro-attracteur, il a été montré que l'alcoylation dont l'allylation est un cas particulier ne marchait quasiment pas. Cela rend d'autant plus surprenants les résultats obtenus par mise en oeuvre de l'invention.

Toutefois, les composés de la colonne V B peuvent également être présents sous la forme d'oxydes trivalents, tels que l'oxyde arsenieux, lesquels ne sont pas exclusifs des composés précédents et donnent de meilleurs résultats en ne défavorisant pas la monoallylation .

En général, ces composés augmentent significativement la cinétique de la réaction. Dans ce cas, ledit élément de la colonne V B est en général de l'arsenic.

Avantageusement, ledit catalyseur comporte comme composés des éléments de la colonne V une trialcoylphosphine, de préférence une triphénylphosphine. Ladite phosphine et ledit métal de la colonne VIII sont avantageusement sous forme de tetrakis phosphine métal.

Dans le cadre de la présente invention, on peut utiliser un catalyseur métallique sous forme élémentaire (degré d'oxydation zéro) ou sous forme oxydée. Ces catalyseurs peuvent se présenter sous forme de sels, d'oxydes ou de complexes. Parmi les sels, oxydes et complexes des métaux précédemment cités, à l'état d'oxydation II, on peut mentionner les chlorures de palladium, l'acétate de palladium, le chlorure de palladium complexé par le benzonitrile. Il convient de souligner que les co-anions sont de faible importance, seuls les cations comptent.

Parmi les complexes des métaux à l'état d'oxydation zéro, on peut citer le palladium dibenzylidèneacétone.

Pour une meilleure mise en oeuvre de l'invention, on préfère utiliser une quantité de catalyseur telle que le rapport molaire entre le catalyseur métallique et les composés des éléments de la colonne V, lorsque ces derniers composés sont sous forme d'agents de coordination, souvent désignés par l'expression anglo-saxonne de ligand, soit compris entre 2 et 100, de préférence de 4 à 30. Le rapport molaire entre les oxydes de la colonne V et le catalyseur métallique est en général compris entre 1/100e à 100, avantageusement de 1/20e à 10, et est de préférence à environ 1. La réaction peut avoir lieu dans, et le réactif comporter, tout solvant, y compris ceux pouvant servir de solvant A ou B ( cf ci-après), tel que notamment :

- le benzène et les solvants aromatiques,

- les alkanes ayant au moins 6 atomes de carbone,
- l'eau,
- l'acétonitrile,
- le benzonitrile,
- le diméthylformamide,
- la N méthylpyrrolidone,
- le nitrobenzène.

Cependant, notamment lorsque l'on opère en solvant non protique notamment non aqueux et/ou lorsque le dérivé allylique comporte un ether il est préférable d'opérer dans des solvants peu basique ayant un indice donneur inférieur à celui du tetrahydrofuranne (20) avantageusement au plus égal à celui de l'éther diéthylique, de préférence à 10, plus préférentiellement à 5. Le caractère inhibant des solvants peut toutefois être utilisé pour accroître la sélectivité des oligo et mono allylation, de préférence la quantité en mole inférieur à celle du substrat.

Il est dans ce cas souhaitable d'éviter les solvants dont les 4 derniers de la liste sont donné à titre d'exemple (ou plus précisement de paradigme dans le sens classique et grammatical du terme). L'absence de solvant ou l'utilisation du substrat ou d'un composant du réactif peut alors être une bonne solution. Il convient de rappeler que l'indice donneur parfois designé par son sigle anglosaxon DN est défini par la valeur de la variation d'enthalpie delta H due à l'association du solvant avec le pentachlorure d'antimoine en milieu chlorure de methylène dilué.

La quantité de solvant utilisée est telle que la concentration du métal de la colonne VIII soit avantageusement supérieure à $10^{-5}$, de préférence de $10^{-2}$ à $10^{-3}$ M dans le solvant. Cette préférence vaut pour la phase aqueuse en cas d'utilisation de cette dernière seule ou en biphasé.

Certaines mises en oeuvre de la présente invention sont particulièrement intéressantes, notamment celles sans solvant ou dont le substrat est le solvant, celles dont le solvant est l'agent allylant, notamment le dérivé allylique, et celles dont le solvant est une phase aqueuse.

Une possibilité particulièrement intéressante est d'utiliser comme pnictines des pnictines hydrosolubles et d'utiliser un réactif aqueux.

Pour rendre hydrosolubles les agents de coordination et notamment les pnictines, il convient de greffer des groupes hydrosolubilisants polaires en faisant attention de ne pas créer de cette manière des nucléophiles qui seraient susceptibles d'interférer avec la réaction.

On peut greffer des groupement neutres tels que les polyols, mais compte tenu de la forte lipophilicité des pnictines, il est préférable que les groupes greffés soient ioniques, cationique comme les ammoniums quaternaires ou anioniques, comme tout groupe constituant la base associée des acides de préférence forts. Dans ce dernier cas, on peut citer les groupes carboxyliques sulfoniques, ou phosphoniques.

On peut notamment citer les groupes utilisés pour modifier les phosphines pour obtenir celles visées dans le brevet français déposé sous le n° 76/22824 et publié sous le n° 2.366.237 ou dans le brevet français publié sous le n° 2.549.840.

A titre de paradigme des phosphines hydrosolubles, on peut citer les triphénylphosphinetrisulfonates $P(C_6H_4-SO_3^-)_3$ solubles, par exemple alcalins et celles de formule $P(C_6H_4-CO_2H)_3$, de préférence sous forme anionique.

Ainsi, selon une mise en oeuvre particulièrement intéressante de la présente invention, on peut utiliser un système biphasique dans lequel une des deux phases liquides est une phase aqueuse dans laquelle le métal de la colonne VIII est solubilisé en phase aqueuse par une phosphine, ou équivalent, hydrosoluble. Cette technique facilite grandement la récupération et le recyclage du catalyseur, lequel recyclage est un des paramètres-clés de la rentabilité de ce type de procédé en raison du prix toujours accru des métaux de la mine du platine. Il est d'ailleurs particulièrement intéressant que les rendements ne soient pas significativement altérés par la mise en oeuvre de deux phase liquides.

Sans que l'on ait pu le prévoir, l'utilisation d'une phase aqueuse permet d'accélérer considérablement la monoallylation sans pour autant accélérer la poly- et notamment la biallylation. Il est ainsi beaucoup plus facile de mener une oligoallylation sélective par rapport à une polyallylation. Lorsque la sélectivité apportée par l'utilisation de la phase aqueuse n'est pas jugée suffisante, on peut pour ce faire jouer sur l'excès de dérivé allylique par exemple d'éther allylique, ou dérivé, par rapport au substrat, par exemple en limitant l'excès stoechiomètrique par rapport à la réaction désirée à 1/2, avantageusement à 1/4, de préférence à 1/10 ou bien en travaillant avec un excès de substrat.

Selon une mise en oeuvre particulièrement intéressante dans la présente invention, on peut utiliser un système bi- ou multiphasique dans lequel une des phases liquides est une phase aqueuse. Notamment lorsque le substrat, le dérivé allylique tel l'éther allylique et/ou le produit d'arrivée sont peu solubles en phase aqueuse, il est possible de mener la réaction, soit sans adjonction de solvant en un système multiphasé, soit en ajoutant un tiers solvant B, soit en ajoutant un solvant A, soit en ajoutant un tiers solvant B et solvant A .

Les solvants A sont des solvants organiques choisis de manière qu'ils dissolvent au moins 1 %, de préférence au moins 2 %, en masse du substrat et sont suffisamment hydrophobes pour n'être pas miscibles à l'eau en toute proportion.

Il est préférable que l'eau ne puisse dissoudre qu'au plus 10 % de solvant A, avantageusement au plus 1 %, en masse et ce même en présence du substrat en tant que tiers solvant.

Il est préférable que le solvant A ne puisse dissoudre qu'au plus 10 % d'eau, avantageusement au plus 1 %, en masse et ce même en présence du substrat en tant que tiers solvant.

Les solvants A peuvent être des mélanges, y compris des fractions pétrolières. Naturellement, dans les conditions opératoires les solvants A doivent être inertes vis-à-vis des substrats et des réactifs utilisés.

Les familles préférées de solvants A sont choisies dans le groupe constitué par les hydrocarbures, les dérivés aromatiques, les éthers, les esters et les solvants halogénés. Ces solvants doivent être moins nucléophiles que les substrats, de manière à ne pas interférer avec la réaction à moins que le substrat soit en excès pour jouer un rôle de solvant.

A titre de paradigmes de ces familles, on peut citer comme dérivés aliphatiques halogénés le dichlorométhane, le dichloro-1,2-éthane, le trichloro-1,1,1- éthane, comme dérivés aromatiques le toluène et comme dérivés aromatiques halogénés le chlorobenzène, comme esters l'acétate d'éthyle et l'acétate d'isopropyle, comme éthers, l'oxyde de tertiobutyle et de méthyle ainsi que l'anisole et les alcools lourds, c'est-à-dire répondant aux contraintes d'immiscibilité comme spécifié ci-dessus.

Pour des raisons d'économie industrielle, il est préférable que le solvant A soit distillable sous pression atmosphérique ou sous vide primaire ou secondaire.

Si les substrats ne sont pas hydrosolubles, il est alors possible de rajouter au solvant A un tiers solvant B, lesquels peuvent être des mélanges, dont le rôle sera de solubiliser le substrat et/ou, le cas échéant, le dérivé allylique ou l'éther allylique, dans la phase aqueuse et qui se partagera entre phases aqueuse et organique lorsque cette dernière existe initialement.

Il est préférable que l'eau puisse dissoudre au moins 1/10 de tiers solvant B, avantageusement au moins 1/3, en masse et ce même en présence du catalyseur avec ses agents de coordination.

Avantageusement, on ajoute le tiers solvant en quantité suffisante pour que la quantité de substrat, et/ou le cas échéant l'éther allylique, ou autre dérivé allylique, soluble dans la phase aqueuse soit au moins du même ordre de grandeur (c'est à dire à un facteur dix près) que la quantité de catalyseur présent dans la phase aqueuse en début de réaction.

Sous réserve des préférences ci-dessus, parmi les solvants utilisables comme tiers solvants, on peut citer les solvants hydrosolubles de types alcool, nitrile, éther (surtout cyclique), acide, sulfone, sulfoxyde, amide, phosphate d'alcoyle(s), phosphonate d'alcoyle(s), phosphinate d'alcoyle(s), oxyde de phosphine, cétone, voire amine non nucléophile notamment par effet stérique.

On peut également choisir le solvant A de manière qu'il joue également le rôle de tiers solvant B. Dans ce cas on utilise des solvants présentant une fonction polaire du type de celle des tiers solvants et ayant une chaîne lipophile choisie de manière que l'eau dissolve ledit tiers solvant à raison d'environ 1/100 à 1/10 en masse.

Le solvant A et/ou le solvant B selon les cas, peut être l'éther allylique ou dérivé.

On ne s'écartera de l'invention, ni en remplaçant ledit dérivé allylique par un des dérivés de l'alcool allylique susceptible de donner un éther ou un alcool in situ dans les conditions de la réaction d'allylation, ni en utilisant comme source du radical allyle un mélange d'éther et/ou d'alcool allylique avec d'autres précuseurs du radical allyle.

Un autre but de la présente invention est un réactif d'allylation utilisant un réactif aussi peu cher que les éthers allyliques et notamment utilisant le réactif précédent.

Ce but est atteint au moyen du réactif d'allylation caractérisé par le fait qu'il comporte :

- une phase aqueuse ;
- un alcool allylique ou un de ses dérivés ;
- un catalyseur comportant au moins un élément de la colonne VIII de la classification périodique des éléments, ledit élément de la colonne VIII de la classification périodique étant maintenu dans la phase aqueuse par complexation avec au moins un agent de coordination (ou ligand) hydrosoluble.

Ce réactif peut comporter ou ne pas comporter de solvant.

Dans le premier cas il peut comporter un solvant A non miscible en totalité avec ladite phase liquide aqueuse.

il peut comporter un solvant B assurant dans la phase aqueuse une solubilité minimale des substrats lipophiles.

- le dérivé allylique alcool ou éther allylique peut présenter la formule (III) suivante:

$$Z\text{-}O\text{-}C(R_1)(R_2)\text{-}C(R_3){=\!=}C(R_4)(R_5)$$

où

- . $R_1$ représente un hydrogène ou un radical alcoyle, de préférence à 1 ou 2 atomes de carbone ;
- . $R_2$ représente un hydrogène ou un radical alcoyle, de préférence à 1 ou 2 atomes de carbone ;
- . $R_3$ représente un hydrogène ou un radical alcoyle ;
- . $R_4$ représente un hydrogène ou un radical alcoyle ;
- . $R_5$ représente un hydrogène ou un radical alcoyle, et Z représente un hydrogène ou un reste déduit d'un alcool par enlèvement d'un groupe hydroxyle ;

le nombre total de carbone du dérivé allylique étant avantageusement au plus égal à 50 de préférence à 30 atomes de carbone (ici le terme alcool doit être compris "lato sensu" et englobe les phénols) par enlèvement d'un groupe hydroxyle.

Ledit alcool allylique ou un de ses dérivés peut être avantageusement choisi parmi les dérivés de formule (III') suivante :

$$Z'\text{-}O\text{-}C(R_1)\,(R_2)\text{-}C(R_3){=\!=}C(R_4)\,(R_5) \qquad \text{(III')}$$

où :R1, R2, $R_3$, $R_4$, et $R_5$ ont la même valeur que précédement et où Z' représente un radical acyle ou de préférence un hydrogène, de préférence d'au plus 10 atomes de carbone.

L'ensemble de l'invention sera plus complètement décrite à l'aide des exemples suivants qui ne doivent en aucun cas être considérés comme limitatifs.

Dans les exemples suivants, on entend par :

TT = le taux de transformation du produit de départ

$$TT = \frac{\text{nombre de moles de produit transformé}}{\text{nombre de moles de produit initial}}$$

RR = le rendement sur le produit initial

$$RR = \frac{\text{nombre de moles de produit final}}{\text{nombre de moles de produit initial}}$$

RT = le rendement sur le produit transformé

$$RT = \frac{\text{nombre de moles de produit final}}{\text{nombre de moles de produit transformé}}$$

MODE OPERATOIRE GENERAL

Les réactifs, solvants et catalyseurs sont introduits en proportions indiquées dans le contenant où la réaction aura lieu.

Cette opération est réalisée dans une boîte à gants mise sous azote, les ingrédients étant préalablement dégazés pour éliminer l'oxygène. Lorsque le point d'ébullition du mélange réactionnel est supérieur à la température testée, on utilise soit un réacteur, soit un tube scellé ou un tube Schott. Lorsque cela n'est pas précisé autrement, on utilise un tube scellé.

Lorsque la température est supérieure au point d'ébullition, on utilise alors un des deux tubes. Lorsque cela n'est pas précisé autrement, on utilise un tube scellé. Les autres conditions réactionnelles sont précisées dans les exemples suivants. Sauf indications contraires, les réactifs utilisés sont les suivants :

- alcool allylique ;
- palladium ;
- tri phénylphosphine.

EXEMPLE 1

Réactifs

m-TFMA : 2 mmol (m-TFMA = meta-trifluorométhyl aniline)

$Pd(P\phi_3)_4$ : 2 % molaire
4 h à 90° C avec agitation
1 équivalent éther allylique : 2 mmol

| RESULTATS | | | | |
|---|---|---|---|---|
| $As_2O_3$ | TT éther allylique | TT (m-TFMA) | RT (N-allyl) | RT (Di-allyl) |
| 0 % | 49 | 46 | 87 | 16 |
| 5 % | 91 | 100 | 20 | 79 |

EXEMPLE 2

m - TFMA : 2 mmol
$Pd(P\phi_3)_4$ : 2 % molaire
4 h à 90° C avec agitation
1/2 équivalent éther allylique : 1 mmol

| RESULTATS | | | | |
|---|---|---|---|---|
| $As_2O_3$ | TT-éther allylique | TT (m-TFMA) | RT (N-allyl) | RT (Di-allyl) |
| 0 % | 89 | 68 | 80 | 12 |
| 4,5 % | 99 | 79 | 78 | 21 |

EXEMPLE 3 - Etude de $Pd(P\phi_3)_4$ et $Pt(P\ \phi_3)_4$

Réactifs

m-TFMA : 2 mmol
Ether allylique : 1 mmol
Catalyseur : 2 % molaire
4 h à 90° C avec agitation

| RESULTAT | | | | |
|---|---|---|---|---|
| catalyseur | TT éther allylique | TT (m-TFMA) | RT (N-allyl) | RT (Diallyl) |
| $Pt\ (P\phi_3)_4$ 4,1 % | 32 | 14 | 74 | 12 |
| $Pd\ (P\phi_3)_4$ 4,5 % | 89 | 68 | 80 | 12 |

Commentaire

Le Pd(O) est nettement plus réactif que Pt(O) pour une même sélectivité.

EXEMPLE 4 - Influence de $B_2\ O_3$

REACTION

$$\text{m-TFMA + éther allylique} \rightarrow \text{N allyl TFMA + biallyl TFMA}$$

Réactifs

m-TFMA : 2 mmol
$Pd(P\phi_3)_4$ : 2 % molaire

4 h à 90° C avec agitation
Ether allylique : 6,5 mmol

| RESULTAT | | | | |
|---|---|---|---|---|
| $B_2O_3$ | TT éther allylique | TT (m-TFMA) | RT (N-allyl) | RT (Diallyl) |
| 5 % | 69 | 100 | 2 | 99 |

EXEMPLE 5 - Réactivité comparée éther et alcool allylique

REACTION

m-TFMA + propénol-1 → N allyl TFMA + N,N-diallyl TFMA

Propénol-1 = alcool allylique.

Réactifs

m-TFMA : 2 mmol
$Pd(P\Phi_3)_4$: 2 %
$As_2O_3$ : 1,1 %
1 h à 90° C

| RESULTAT | | | |
|---|---|---|---|
| Agent allylant | TT (m -TFMA) | RT (N-allyl) | RT (Diallyl) |
| Alc.allylique 11,5 mmol | 78 | 65 | 31 |
| Eth. allylique 11,5 mmol | 99 | 19 | 82 |

D'autre part, on a dosé alcool et éther allylique :
TT (alcool allylique) = 74 RT (éther allylique) = 31
TT (éther allylique) = 35 RT (alcool allylique) = 2

EXEMPLE 6 - Réactivité comparée éther et alcool allylique

Réactifs

m -TFMA : 2,1 mmol
$Pd(P\phi_3)_4$ : 2,2 %
15 mn à 90° C

| RESULTAT | | | |
|---|---|---|---|
| Agent allylant | TT (m -TFMA) | RT (N-allyl) | RT (Di-allyl) |
| alcool allylique 2,1 mmol | 8 | 61 | 26 |
| éther allylique 1 mmol | 7 | 50 | 20 |

D'autre part, on a dosé alcool et éther allylique :
TT (alcool allylique) = 19 RT (éther allylique) = 3 TT (éther allylique) = 5 RT (alcool allylique) = 5

EXEMPLE 7 - m-TFMA Allylation avec éther allylique O + $Pt^{II}$ + tPPTS

Dans un tube Schott de 30 cc :

- $H_2O$ - 5,109 g
- $PtCl_2$ (COD) ; PM = 374 ; 56,1 mg ; 0,15 mmole COD = 1,5 -cyclooctadiene
- TPPTS (0,522 mmole/g) : 1,6458 g (0,859 mmole) dans l'eau. (TPPTS = triphényl phosphine trisulfonate de sodium)
- m-TFMA : 1,1442 g ; PM = 161,1 ; 7,1024 mmole
- éther allylique : PM = 98,15 : 1,5613 g ; 15,907 mmole

$$\frac{\text{Rapport éther allylique}}{\text{m-TFMA}} = 2,24 \qquad \frac{PtCl_2(COD)}{\text{m-TFMA}} = 2,1\ \%$$

$$\frac{TPPTS}{PtCl_2(COD)} = 5,73$$

Durée de la réaction = 1h 30 à 90°C, puis on refroidit à température ambiante, on extrait à l'acétate d'éthyle puis on décante.

L'analyse est réalisée en chromatographie phase gazeuse sur la phase organique et donne les résultats suivants.

TT = 18,5 %
N-allyl : RT = > 95 %
Di-allyl : RT < 0,2 %

EXEMPLE 8 - Influence de $As_2O_3$

Réactifs

Phénol : 2 mmol
$Pd(P\phi_3)_4$ : 2 % molaire
4 h à 90° C avec agitation
1 équivalent d'éther allylique : 2 mmol

| RESULTAT | | | | |
|---|---|---|---|---|
| $As_2O_3$ | TT-éther allylique | TT (phénol) | RT (O-allyl) | RT (C-allyl) |
| 0 % | 61 | 39 | 18 | 41 |
| 5 % | 70 | 59 | 23 | 26 |

EXEMPLE 9 - Influence du catalyseur

Réactifs

Phénol : 2 mmol
$Pd(P\phi_3)_4$ ou $Pt(P\phi_3)_4$ : 2 % molaire
4 h à 90° C avec agitation
1/2 équivalent : 1 mmol

| RESULTAT | | | | | |
|---|---|---|---|---|---|
| Catalyseur 2 % | $As_2O_3$ | TT | TT (phénol) | RT (O-allyl) | RT (C-allyl) |
| Pt $(P\phi_3)_4$ | 4,2 % | 29 | 12 | 60 | 13 |

(suite)

| RESULTAT | | | | | |
|---|---|---|---|---|---|
| Catalyseur 2 % | $As_2O_3$ | TT | TT (phénol) | RT (O-allyl) | RT (C-allyl) |
| Pd ($P\phi_3$)$_4$ | 4,5 % | 68 | 38 | 30 | 28 |

Exemple 10 (monophasé organique)

Réactifs

m-TFMA : 2 mmol
Alcool allylique : 11,2 mmol
Pd ($P\Phi_3$)$_4$ : 2 % molaire
1 h à 90° C avec agitation

| RESULTAT | | | |
|---|---|---|---|
| mTFMA | | RT | RT |
| | TT | MONO | DI |
| | 31 | 80 | 13 |

EXEMPLE 11 biphasé (organique - aqueuse)

Réactifs

| | |
|---|---|
| $H_2O$ : | 5 ml |
| Alcool allylique : | 3,3 ml (66 mmol) |
| m-TFMA : | 2ml (16 mmol) |
| TPPTS : | solution aqueuse 0,522 mmol/g j T = 90° C |

| CATALYSEUR (mmol) | TPPTS Métal Rapport molaire | durée (h) | TT (%) | RR (%) | |
|---|---|---|---|---|---|
| | | | | N-allyl | N-diallyl |
| $Pd(OAc)_2$ (0,16) | 5,2 | 0,75 | 81 | 80 | 1 |
| $PtCl_2$(COD) (0,12) | 6,5 | 5,5 | 94 | 76 | 18 |
| RhCl(COD)/$_2$ (0,19) | 4,5 | 3,5 | 74 | n.d. | n.d. |
| $RuCl_3$ (0,16) | 5,4 | 3,5 | 95 | 78 | 17 |
| $NiCl_2/NaBH_4$ (0,2) | 4,6 | 2,5 | 8 | 8 | - |
| n.d. = produit réactionnel identifié mais rendement non mesuré. | | | | | |

Commentaire

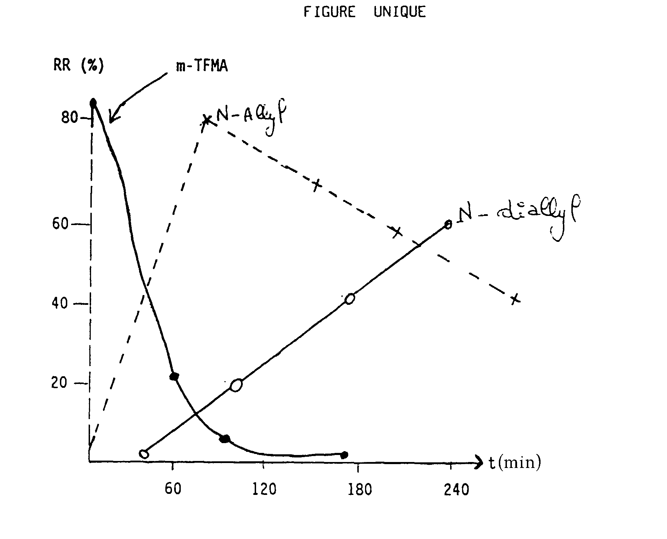

Pd : La première allylation est très rapide. Des prélèvements de la phase organique ont permis de l'apprécier (cf. Figure unique).
Pt : Même profil réactionnel que ci-dessus mais en moins actif.
Ni :La réduction de $NiCl_2$ par le borohydrure de sodium en présence de TPPTS conduit à la formation de Ni(TPPTS)$_4$ de couleur caractéristique rouge.

EXEMPLE 12

Système biphasique organique/aqueux

Réactifs

| | |
|---|---|
| Anilide (métatrifluorométhylacétanilide) : | 1,08 g (5,32 mmoles) |
| Alcool allylique/anilide : | 5,92 |
| Alcool allylique : | 1,83 g (31,5 mmoles) |
| $Pd^{II}Ac_2$ : | 70 mg ; PM = 224,49 ; 0,31 mmoles ; $Pd^{II}$/anilide = 5,86 % |
| TPPTS (0,522 mmoles/g) : | 2,39 g (1,2476 mmoles) |
| $H_2O$ : | 2,35 g |
| TPPTS/$Pd^{II}$ : 16 h 30 à 139° C - 140° C | 4,05 |
| Anilide : | TT = < 10 % |
| Anilide allylée : | RR = 7 % |

EXEMPLE 13 (comparatif)

Système biphasique organique/aqueux : rôle de l'insaturation allylique Essai avec du propanol-1

Réactifs

| | |
|---|---|
| Aniline (métatrifluorométhylaniline) : | 1,2 g (7 mmoles) |
| $PtCl_2$ : | 53 mg ; PM = 374 ; 0,14 mmoles |
| TPPTS (0,522 mmoles/g) : | 1,6 g (0,84 mmoles) |
| $H_2O$ : | 5,1 g |
| Propanol-1 : | 2,2 g (37 mmoles) |
| Durée de la réaction : | 1 h 48 à 90° C |

Le mélange réactionnel est repris à l'acétate d'éthyle et cette dernière phase est soumise à un dosage par chromatographie en phase gazeuse :

| | |
|---|---|
| Aniline : | TT = < 3 % |
| Aniline propylée : | RR = 0 % |

De ce résultat on peut déduire la nécessité de la présence de l'insaturation allylique.

EXEMPLE 14

CATALYSE PAR LE SYSTEME = TETRAKIS TRIPHENYL - PHOSPHINE PALLADIUM (0)-ANHYDRIDE ARSENIEUX

- Amide : 4 mmol.
- Alcool allylique : 1,5 ml (22 mmol).
- $Pd(P\phi_3)_4$ : 2 % (0,08 mmol).
- $As_2O_3$ : 4 % (0,16 mmol).
- Manipulations faites sous atmosphère inerte.
- Ar = m $CF_3\Phi$-

| AMIDE | CATALYSEUR | CONDITIONS | TT(amide) (%) | RT (%) |
|---|---|---|---|---|
| $ArNHCOCH_3$ | $Pd(PO_3)_4/As_2O_3$ | 90°C - 17 h | 22 | 100 |
| $ArNHCOCH_3$ | $Pd(P\phi_3)_4$ | 140°C - 17 h | 88 | 90 |
| $ArNHCOCH_3$ | $Pd(P\phi_3)_4/As_2O_3$ | 140°C - 17 h | 70 | 89 |
| $ArNHCOCHCl_2$ | $Pd(P\phi_3)_4$ | 140°C - 17 h | 86,5 | traces |

- a) Réaction effectuée dans un réacteur de 30 ml.

- b) Réaction effectuée dans un tube scellé.

EXEMPLE 15

Utilisation de Pt(0)

REACTION

N-dichloroacétyl m T.F.M.A + alcool allylique $\xrightarrow{cat.}$ NAND + $H_2O$

où
NAND = N-allyl, N-dichloroacétyl, méta-trifluorométhylaniline
m T.F.M.A = méta trifluorométhylaniline.

Réactifs

N-dichloroacétyl m T.F.M.A : 2 mmol
Alcool Allylique : 13 mmol
$Pt(P\phi_3)_4$ : 2 % molaire
4 h à 90° C avec agitation

| RESULTATS | | |
|---|---|---|
| $As_2O_3$ | TT (Dichloro) | RT (NAND) |
| 0 % | 11 | 80-100 |
| 8,7 % | 14 | 68 |

Commentaires :

. La réaction a lieu avec une réactivité relativement faible, mais une bonne sélectivité. $As_2O_3$ n'a que peu d'influence.

EXEMPLE 16

Réactifs

Acétanilide : 2 mmol
Alcool allylique : 12 mmol
$Pd(P\Phi_3)_4$ : 2,3 % molaire
4 h à 90° C avec agitation

| RESULTATS | | |
|---|---|---|
| REACTIF | TT | RT (estimé) |
| p-méthoxy acétanilide | 66 | 10 |
| acétanilide | 31 | 75 |

EXEMPLE 17

ROLE DU CHLORURE STANNEUX

Réactifs

m-TFMA : 2 mmol
$PtCl_2$ : 2 % molaire (0,08 mmol)
4 h à 90° C avec agitation

Excès d'alcool allylique qui joue aussi le rôle de solvant ou 1 équivalent dissous dans le diglyme $SnCl_2$ est ajouté en quantité variable.

| RESULTATS | | | | |
|---|---|---|---|---|
| rapport mol Sn/Pt % | solvant | TT (%) (m-TFMA) | RT (N-allyl) | RT (Di-allyl) |
| 0 | alc. all. | 92,5 | 79 | 6 |
| 1 | alc. all. | 96,5 | 72 | 18 |
| 2 | alc. all. | 100 | 29,5 | 68,5 |
| 0 | diglyme | 22 | 66 | 1 |
| 2 | diglyme | 70 | 77 | 7,5 |
| 3 | diglyme | 76 | 73,5 | 13,5 |

Dans tous les cas, la présence de chlorure d'étain nuit à la monoallylation bien que les rendement soient sensiblement améliorés. A noter l'effet défavorable du diglyme sur la réaction mais relativement favorable sur la sélectivité.

EXEMPLE 18

PLATINE A UN AUTRE DEGRE D'OXYDATION ET SANS PNICTINE

Les réactions sont faites à 70° C pendant 3 h.

m-TFMA = 4 mmol
Catalyseur = 2 % (0,08 mmol)

| Catalyseur 2 % (mol) | TT (mTFMA) (%) | RT N allyl (%) | RT Diallyl (%) |
|---|---|---|---|
| $H_2PtCl_6$ | 57,5 | 38 | 0 |

Cet exemple montre que des agents de coordination du type halogénure surtout des 3 premières périodes suffisent notamment pour le platine.

EXEMPLE 19

CATALYSE PAR LE PALLADIUM (0)

| Essai | Catalyseur 2 % (mol) | TT(mTFMA) (%) | RT N allyl (%) | RT Diallyl (%) |
|---|---|---|---|---|
| 169-32 | $Pd(P\phi_3)_4$ | 22 | 83 | 5 |

Essai effectué à 70° C pendant 3 h en utilisant 4 mmol de m-TFMA, 4 mmol d'alcool allylique et 1,2 ml de diglyme. Afin de préserver les qualités du catalyseur, le réacteur est chargé sous atmosphère inerte dans une boîte à gants. Dans les mêmes conditions opératoires, le $PtCl_2$ donnait pour TT = 22 %, RT N allyl = 66 % et RT Diallyl = 1 %. L'activité du palladium (0) peut donc être comparée à celle du platine (II) avec un léger gain en composé N-allylé.

EXEMPLE 20

INFLUENCE DE L'ANHYDRIDE ARSENIEUX

| Essai | Catalyseur(% mol) | TT(m-TFMA) (%) | RT N-allyl (%) | RT Diallyl (%) |
|---|---|---|---|---|
| 150-18 (a) | $As_2O_3$ 2 % | 9 | 0 | 0 |
| 161-28 (a) | $Pd(P\phi_3)_4$ 2 % $As_2O_3$ 4 % | 100 | 5,5 | 95,5 |
| 169-31 (b) | $Pd(P\phi_3)_4$ 2 % $As_2O_3$ 4 % | 75,5 | 55 | 40,5 |
| 169-32 (b) | $Pd(P\phi_3)_4$ 2% | 22 | 83 | 5 |
| 151-20 (b) | $PtCl_2$ 2 % $As_2O_3$ 4 % | 13,5 | 45 | 0 |
| 143-7 | $PtCl_2$ 2 % | 22 | 66 | 1 |

a) : m.TFMA = 4 mmol, alcool allylique = 22 mmol, 70° C, 3 h.
(b) : m.TFMA = 4 mmol, alcool allylique = 4 mmol, diglyme 1,2 ml, 70° C, 3 heures.

On constate que le gain de réactivité apporté par l'ajout de l'anhydride arsénieux dans le milieu réactionnel, est observé avec le palladium (0).

EXEMPLE 21

ROLE DES ELEMENTS DE LA COLONNE V

Réactifs

m-TFMA : 2 mmol
Alcool allylique : 11,2 mmol ; Pt ou Pd $(P\Phi_3)_4$ : 2 % molaire
Cocatalyseur 4 % molaire
4 h à 90° C avec agitation magnétique

| RESULTATS | | | |
|---|---|---|---|
| Catalyseur | TT | RT MONO | RT DI |
| Pd | 35 | 70 | 8 |
| Pt | 68 | 67 | 28 |
| $Pd/As_2O_3$ | 99 | 14 | 89 |
| $Pt/As_2O_3$ | 100 | 1 | 99 |
| $Pd/Sb_2O_3$ | 22 | 74 | 9 |
| $Pt/Sb_2O_3$ | 55 | 64 | 13 |

EXEMPLE 22

INFLUENCE DE COCATALYSEURS

Réactifs

m-TFMA : 2 mmol
Alcool allylique : 11,2 mmol
Pd $(P\Phi_3)_4$ : 2 % molaire
Cocatalyseur : 4 % molaire
4 h à 90° C avec agitation magnétique

| RESULTATS | | | |
|---|---|---|---|
| COCATA | TT | RT MONO | RT DI |
| $Bi_2O_3$ | 22 | 82 | 7 |
| $B_2O_3$ | 74 | 70 | 31 |

EXEMPLE 23

ROLE DU SUBSTRAT

Réactifs

Aniline 2 mmole
Alcool allylique 11,2 mmol
Pd $(P\Phi_3)_4$ 2 % molaire
4 h à 90° C avec agitation

| RESULTATS | | | |
|---|---|---|---|
| REACTIF | TT | RT MONO (estime) | RT DI (estime) |
| p-méthoxyaniline | 97 | 12 | 88 |
| ANILINE | 97 | 0,2 | 66 |
| p-nitroaniline | 13 | 95 | 0 |
| m-TFMA | 35 | 70 | 8 |

EXEMPLE 24

Réactifs

Aniline : 2 mmol
Alcool allylique : 11,2 mmol
Pd $(P\Phi_3)_4$ : 2 % molaire
$As_2O_3$ : 4 % molaire
4 h à 90° C avec agitation

| RESULTATS | | | |
|---|---|---|---|
| REACTIF | TT | RT MONO (estime) | AT DI (estime) |
| p-méthoxyaniline | 100 | 1 | 100 |
| aniline | 99 | 0,6 | 90 |

(suite)

| RESULTATS | | | |
|---|---|---|---|
| REACTIF | TT | RT MONO (estime) | AT DI (estime) |
| p-nitroaniline | 40 | 100 | 0 |
| m-TFMA | 99 | 14 | 89 |

EXEMPLE 25

CINETIQUE

Réactifs

m-TFMA (métatrifluorométhylaniline): 2 mmol
Alcool allylique : 11,2 mmol
$Pd(P\Phi_3)_4$ : 2 % molaire
$As_2O_3$ : 5 % molaire

| RESULTATS | | | |
|---|---|---|---|
| m-TFMA | TT | RT MONO | RT DI |
| 4 h . 90° C | 99 | 14 | 89 |
| 3 h . 90° C* | 100 | 6 | 100 |
| 2 h . 90° C | 100 | 19 | 93 |
| 1 h . 90° C | 100 | 2 | 100 |

* (4 % $As_2O_3$)

EXEMPLE 26

REDUCTION DE LA TEMPERATURE

Réactifs

m-TFMA (métatrifluorométhylaniline) : 2 mmol
Alcool allylique : 11,2 mmol
$Pd(P\Phi_3)_4$ : 2 % molaire
$As_2O_3$ : 4 % molaire

| RESULTATS | | | |
|---|---|---|---|
| M-TFMA | TT | RT MONO | RT DI |
| 1 h . 90. C * | 100 | 2 | 100 |
| 1 h . 80. C | 100 | 11 | 87 |
| 1 h . 70. C | 99 | 12 | 89 |
| 1 h . 60. C | 99 | 10 | 90 |

* ($As_2O_3$ 5,4 %)

**Revendications**

**1.** Réactif d'allylation comportant :

- une phase aqueuse ;
- un alcool allylique ou un dérivé allylique;
- un catalyseur comportant au moins un élément de la colonne VIII de la classification périodique des éléments, ledit élément de la colonne VIII de la classification périodique étant maintenu dans la phase aqueuse par complexation avec au moins un agent de coordination (ou ligand) hydrosoluble; avec la condition que lorsque l'agent de coordination est associé à un nitrile, ledit dérivé allylique n'est ni un ester de l'acide carbonique, ni un ester de l'acide acétique.

**2.** Réactif selon la revendication 1, caractérisé par le fait que ledit réactif comporte en outre un solvant A non miscible en totalité avec ladite phase liquide aqueuse.

**3.** Réactif selon l'une des revendications 1 à 2, caractérisé par le fait qu'il comporte en outre un solvant assurant une solubilité minimale des substrats lipophiles.

**4.** Réactif selon l'une des revendications 1 à 3, caractérisé par le fait que ledit alcool allylique ou un dérivé allylique est choisi parmi les dérivés de formule (III) suivante :

$$Z\text{-}O\text{-}C(R_1)(R_2)\text{-}C(R_3){=\!=}C(R_4)(R_5) \qquad (III)$$

où:

- $R_1$ représente un hydrogène ou un radical hydrocarboné choisi parmi les radicaux alcoyles au sens du dictionnaire de chimie Duval et les radicaux aryles ;
- $R_2$ représente un hydrogène ou un radical hydrocarboné choisi parmi les radicaux alcoyles au sens du dictionnaire de chimie Duval et les radicaux aryles ;
- $R_3$ représente un hydrogène ou un radical hydrocarboné choisi parmi les radicaux alcoyles au sens du dictionnaire de chimie Duval et les radicaux aryles ;
- $R_4$ représente un hydrogène ou un radical hydrocarboné choisi parmi les radicaux alcoyles au sens du dictionnaire de chimie Duval et les radicaux aryles ;
- $R_5$ représente un hydrogène ou un radical hydrocarboné choisi parmi les radicaux alcoyles au sens du dictionnaire de chimie Duval et les radicaux aryles ;

et Z représente un hydrogène ou un reste déduit d'un alcool par enlèvement d'un groupe hydroxyle ;
le nombre total de carbone du dérivé allylique étant au plus égal à 50, avantageusement à 30, atomes de carbone.

**5.** Réactif selon l'une des revendications 1 à 4, caractérisé par le fait que ledit agent de coordination est une pnictine, avantageusement choisie parmi les trialcoylphosphines et les triphénylphosphines rendues hydrosolubles par greffage, de préférence les triphénylphosphine trisulfonates.

**6.** Réactif selon l'une des revendications 1 à 5, caractérisé par le fait que lorsque la molécule à allyler présente au moins un halogène, ledit élément de la colonne VIII est le platine.

**7.** Réactif selon l'une des revendications 5 et 6, caractérisé par le fait que ladite phosphine et ledit métal de la colonne VIII sont sous forme de tetrakis phosphine métal.

**8.** Réactif selon l'une des revendications 1 à 7, caractérisé par le fait que l'élément de la colonne VIII est un métal de la mine du platine, avantageusement le palladium.

**9.** Réactif selon l'une des revendications 1 à 8, caractérisé par le fait que ledit catalyseur comporte en outre un oxyde

d'un élément de la colonne V B de la classification périodique des éléments.

**10.** Réactif selon la revendication 9, caractérisé par le fait que ledit élément de la colonne V B est à l'état trivalent.

**11.** Réactif selon l'une des revendications 9 et 10, caractérisé par le fait que ledit élément de la colonne V B est l'arsenic.

**12.** Réactif selon l'une des revendications 4 à 11, caractérisé par le fait qu'au moins 2, avantageusement 3, des radicaux R1 à R5 ont au plus deux atomes de carbone.

**13.** Réactif selon les revendications 1 à 12, caractérisé par le fait qu'il comporte 2 phases liquides non miscibles dont l'une est ladite phase aqueuse contenant la phosphine hydrosoluble.

**14.** Procédé pour allyler un nucléophile, notamment les phénols et les anilines, caractérisé par le fait que l'on fait réagir ledit nucléophile avec un alcool allylique ou un dérivé allylique en présence d'un catalyseur comportant au moins un élément de la colonne VIII de la classification périodique des éléments, ledit élément étant maintenu dans la phase aqueuse par complexation avec au moins un agent de coordination (ou ligand) hydrosoluble, et par le fait que ledit nucléophile est un composé de formule (V) suivante :

$$(R)_n \text{ Ar-Y-H} \qquad (V)$$

dans laquelle :

- Ar représente un radical aromatique mono ou polycyclique, condensé ou non, homo ou hétérocyclique ;
- R représente au moins un substituant choisi indépendamment parmi le chlore, le brome, les radicaux alkyles linéaires, ramifiés, cycliques saturés ou insaturés éventuellement substitués, éther, alcoxy éventuellement substitué, aryl éventuellement substitué, aryloxy, amino, hydroxy, carboxylate, acyloxy, ester, amido, nitrile, acide ;
- n est égal à 0 ou à un nombre entier égal à 1, 2, 3 ou 4,
- Y représente un atome de chalcogène, de préférence d'oxygène ou un groupement NR' avec R' représentant un acyle ou un alcoyle léger (de 1 à 10, de préférence de 1 à 6 atomes de carbone) ou un radical aryle ou avantageusement un atome d'hydrogène.

avec la condition que lorsque Y est NR', le composé présente une fonction anilide ou une fonction aniline désactivée par la présence d'un groupe électroattracteur et/ou par la présence d'un hétéroatome dans le noyau.

**15.** Procédé selon la revendication 14, caractérisé par le fait que ledit alcool allylique ou un de ses dérivés est choisi parmi les dérivés de formule (III) suivante

$$\text{Z-O-C}(R_1)(R_2)\text{-C}(R_3)\text{==C}(R_4)(R_5) \qquad (III)$$

où:

- $R_1$ représente un hydrogène ou un radical hydrocarboné choisi parmi les radicaux alcoyles au sens du dictionnaire de chimie Duval et les radicaux aryles, de préférence à 1 ou 2 atomes de carbone ;
- $R_2$ représente un hydrogène ou un radical hydrocarbonné choisi parmi les radicaux alcoyles au sens du dictionnaire de chimie Duval et les radicaux aryles, de préférence à 1 ou 2 atomes de carbone ;
- $R_3$ représente un hydrogène ou un radical hydrocarbonné choisi parmi les radicaux alcoyles au sens du dictionnaire de chimie Duval et les radicaux aryles, de préférence à 1 ou 2 atomes de carbone ;
- $R_4$ représente un hydrogène ou un radical hydrocarbonné choisi parmi les radicaux alcoyles au sens du dictionnaire de chimie Duval et les radicaux aryles, de préférence à 1 ou 2 atomes de carbone ;

- $R_5$ représente un hydrogène ou un radical hydrocarbonné choisi parmi les radicaux alcoyles au sens du dictionnaire de chimie Duval et les radicaux aryles, de préférence à 1 ou 2 atomes de carbone ;
- Z représente un hydrogène ou un radical acyle, de préférence d'au plus 10 atomes de carbone.

**16.** Procédé selon l'une des revendications 14 et 15, caractérisé par le fait que ledit composé de formule (V) est choisi parmi les anilines désactivées de formule II suivante :

$$A - Ar - N - H_2 \qquad (II)$$

où :

- A représente un substituant , de préférence un groupe attracteur ou un atome d'hydrogène ;
- Ar a la même signification que précédemment.

**17.** Procédé selon les revendications 14 et 15, caractérisé par le fait que ledit composé de formule (V) est choisi parmi les phénols présentant la formule IV suivante :

$$(R)_n \text{ Ar-O-H} \qquad (IV)$$

où:

- Ar, R et n ont la même signification que précédemment.

**18.** Procédé selon l'une des revendications 14 à 17, caractérisé par le fait que ledit agent de coordination est une pnictine, avantageusement choisie parmi les trialcoylphosphines et les triphénylphosphines rendues hydrosolubles par greffage.

**19.** Procédé selon les revendications 14 à 18, caractérisé par le fait que l'on soumet le nucléophile de formule V à un réactif selon l'une des revendications 1 à 13.

**20.** Procédé selon l'une des revendications 17 à 19, caractérisé par le fait que lorsque le nucléophile de formule V présente au moins un halogène, ledit élément de la colonne VIII est le platine.

**21.** Procédé selon l'une des revendications 19 et 20, caractérisé par le fait que ladite phosphine et ledit métal de la colonne VIII sont sous forme de tetrakis phosphine métal.

**22.** Procédé selon l'une des revendications 14 à 21, caractérisé par le fait que l'élément de la colonne VIII est un métal de la mine du platine, avantageusement le palladium.

**23.** Procédé selon l'une des revendications 14 à 22, caractérisé par le fait que ledit catalyseur comporte en outre un oxyde d'un élément de la colonne V B de la classification périodique des éléments.

**24.** Procédé selon la revendication 23, caractérisé par le fait que ledit élément de la colonne V B est à l'état trivalent.

**25.** Procédé selon l'une des revendications 21 et 22, caractérisé par le fait que ledit élément de la colonne V B est l'arsenic.

**26.** Procédé selon l'une des revendications 14 à 25, caractérisé par le fait que ledit agent de coordination est une phosphine hydrosoluble, de préférence un triphénylephosphine trisulfonate, avantageusement de sodium.

**27.** Procédé selon l'une des revendications 14 à 26, caractérisé par le fait que la réaction a lieu dans un milieu comportant 2 phases liquides non miscibles dont l'une est une phase aqueuse contenant ledit agent de coordination.

**28.** Procédé selon la revendication 27, caractérisé par le fait qu'après la réaction d'allylation, la phase aqueuse est récupérée et réutilisée pour une nouvelle opération d'allylation.

**Patentansprüche**

**1.** Reagenz zur Allylierung, enthaltend:

- eine wäßrige Phase;
- einen Allylalkohol oder ein Allylderivat;
- einen Katalysator mit mindestens einem Element aus der Gruppe VIII des Periodensystems der Elemente, wobei das Element aus der Gruppe VIII des Periodensystems durch Komplexierung mit mindestens einem wasserlöslichen Koordinierungsreagenz (oder Liganden) in der wäßrigen Phase gehalten wird; mit der Maßgabe, daß, wenn das Koordinierungsreagenz an ein Nitril gebunden ist, das Allylderivat weder ein Carbonsäureester noch ein Essigsäureester ist.

**2.** Reagenz nach Anspruch 1, dadurch gekennzeichnet, daß das Reagenz außerdem ein Lösemittel A enthält, das in seiner Gesamtheit mit der flüssigen wäßrigen Phase nicht mischbar ist.

**3.** Reagenz nach Anspruch 1 oder 2, dadurch gekennzeichnet, daß es außerdem ein Lösemittel enthält, das eine minimale Löslichkeit der lipophilen Ausgangsmaterialien gewährleistet.

**4.** Reagenz nach einem der Ansprüche 1 bis 3, dadurch gekennzeichnet, daß der Allylalkohol oder das Allylderivat ausgewählt sind aus Verbindungen der folgenden Formel (III):

$$Z\text{-}O\text{-}C(R_1)(R_2)\text{-}C(R_3){=}{=}C(R_4)(R_5) \qquad \text{III}$$

wobei:

- $R_1$ Wasserstoff oder einen Kohlenwasserstoffrest darstellt, ausgewählt aus Alkylresten im Sinne von Duvals Chemielexikon (Dictionnaire de Chimie Duval) und Arylresten;
- $R_2$ Wasserstoff oder ein Kohlenwasserstoffrest darstellt, ausgewählt aus Alkylresten im Sinne von Duvals Chemielexikon (Dictionnaire de Chimie Duval) und Arylresten;
- $R_3$ Wasserstoff oder einen Kohlenwasserstoffrest darstellt, ausgewählt aus Alkylresten im Sinne von Duvals Chemielexikon (Dictionnaire de Chimie Duval) und Arylresten;
- $R_4$ Wasserstoff oder einen Kohlenwasserstoffrest darstellt, ausgewählt aus Alkylresten im Sinne von Duvals Chemielexikon (Dictionnaire de Chimie Duval) und Arylresten;
- $R_5$ Wasserstoff oder einen Kohlenwasserstoffrest darstellt, ausgewählt aus Alkylresten im Sinne von Duvals Chemielexikon (Dictionnaire de Chimie Duval) und Arylresten; und
- Z Wasserstoff oder einen Rest darstellt, der sich von einem Alkohol unter Entfemung einer Hydroxylgruppe ableitet,

wobei die Kohlenstoffgesamtanzahl der Allylverbindung bei höchstens 50 Kohlenstoffatomen, vorzugsweise bei 30 Kohlenstoffatomen, liegt.

**5.** Reagenz nach einem der Ansprüche 1 bis 4, dadurch gekennzeichnet, daß das Koordinierungsreagenz ein Pnictin ist, vorzugsweise ausgewählt aus mittels Pfropfung wasserlöslich gemachten Trialkylphosphinen und Triphenylphosphinen, insbesondere Triphenylphosphintrisulfonaten.

**6.** Reagenz nach einem der Ansprüche 1 bis 5, dadurch gekennzeichnet, daß, wenn das zu allylierende Molekül mindestens ein Halogen aufweist, das Element aus der Gruppe VIII Platin ist.

**7.** Reagenz nach Anspruch 5 oder 6, dadurch gekennzeichnet, daß das Phosphin und das Metall aus der Gruppe VIII in Form von Tetrakisphosphinmetall vorliegt.

**8.** Reagenz nach einem der Ansprüche 1 bis 7, dadurch gekennzeichnet, daß das Element aus der Gruppe VIII ein Metall aus der Platinmine ist, vorzugsweise Palladium.

**9.** Reagenz nach einem der Ansprüche 1 bis 8, dadurch gekennzeichnet, daß der Katalysator außerdem ein Oxid eines Elementes der Gruppe V B des Periodensystems der Elemente enthält.

**10.** Reagenz nach Anspruch 9, dadurch gekennzeichnet, daß das Element aus der Gruppe V B im dreiwertigen Zustand vorliegt.

**11.** Reagenz nach Anspruch 9 oder 10, dadurch gekennzeichnet, daß das Element aus der Gruppe V B Arsen ist.

**12.** Reagenz nach einem der Ansprüche 4 bis 11, dadurch gekennzeichnet, daß mindestens zwei, vorzugsweise drei, der Reste $R_1$ bis $R_5$ höchstens zwei Kohlenstoffatome aufweisen.

**13.** Reagenz nach einem der Ansprüche 1 bis 12, dadurch gekennzeichnet, daß es zwei nichtmischbare flüssige Phasen aufweist, von denen eine die wäßrige Phase darstellt, die das wasserlösliche Phosphin enthält.

**14.** Verfahren zur Allylierung eines Nucleophils, insbesondere von Phenolen und Anilinen, dadurch gekennzeichnet, daß man das Nucleophil in Gegenwart eines Katalysators, der mindestens ein Element aus der Gruppe VIII des Periodensystems der Elemente enthält, mit einem Allylalkohol oder einem Allylderivat reagieren läßt, wobei das Element durch Komplexbildung mit mindestens einem wasserlöslichen Koordinierungsreagenz (oder Liganden) in der wäßrigen Phase gehalten wird, und daß das Nucleophil eine Verbindung der folgenden Formel (V)

$$(R)_n Ar\text{-}Y\text{-}H \qquad (V)$$

darstellt, in der:

- Ar einen homo- oder heterocyclischen, kondensierten oder nichtkondensierten, mono- oder polycyclischen aromatischen Rest darstellt;
- R mindestens einen Substituenten darstellt, unabhängig ausgewählt aus Chlor; Brom; gegebenenfalls substituierten, gesättigten oder ungesättigten, cyclischen, verzweigten oder linearen Alkylresten; Etherresten, gegebenenfalls substituierten Alkoxyresten; gegebenenfalls substituierten Arylresten; Aryloxyresten; Aminoresten; Hydroxyresten; Carboxylatresten; Acyloxyresten; Esterresten; Amidoresten; Nitrilresten und Säureresten;
- n gleich 0 oder eine ganze Zahl von 1, 2, 3 oder 4 ist;
- Y ein Chalcogen, vorzugsweise Sauerstoff, eine Gruppe NR', wobei R' einen leichten Acyl- oder Alkylrest (mit 1 bis 10 Kohlenstoffatomen, vorzugsweise mit 1 bis 6 Kohlenstoffatomen) darstellt, einen Arylrest oder vorzugsweise ein Wasserstoffatom darstellt;

mit der Maßgabe, daß, wenn Y NR' ist, die Verbindung eine Anilidfunktion oder eine durch die Gegenwart einer elektronenziehenden Gruppe und/oder durch die Gegenwart eines im Kem befindlichen Heteroatoms desaktivierte Anilinfunktion darstellt.

**15.** Verfahren nach Anspruch 14, dadurch gekennzeichnet, daß der Allylalkohol oder seine Derivate ausgewählt sind aus Derivaten der folgenden Formel

$$Z\text{-}O\text{-}C(R_1)(R_2)\text{-}C(R_3){=}{=}C(R_4)(R_5) \qquad III$$

wobei:

- $R_1$ Wasserstoff oder einen Kohlenwasserstoffrest darstellt, ausgewählt aus Alkylresten im Sinne des Chemielexikons von Duval (Dictionnaire de Chimie Duval) und Arylresten, vorzugsweise mit 1 bis 2 Kohlenstoffatomen;
- $R_2$ Wasserstoff oder einen Kohlenwasserstoffrest darstellt, ausgewählt aus Alkylresten im Sinne des Chemielexikons von Duval (Dictionnaire de Chimie Duval) und Arylresten, vorzugsweise mit 1 bis 2 Kohlenstoffatomen;
- $R_3$ Wasserstoff oder einen Kohlenwasserstoffrest darstellt, ausgewählt aus Alkylresten im Sinne des Chemielexikons von Duval (Dictionnaire de Chimie Duval) und Arylresten, vorzugsweise mit 1 bis 2 Kohlenstoffatomen;

- $R_4$ Wasserstoff oder einen Kohlenwasserstoffrest darstellt, ausgewählt aus Alkylresten im Sinne des Chemielexikons von Duval (Dictionnaire de Chimie Duval) und Arylresten, vorzugsweise mit 1 bis 2 Kohlenstoffatomen;
- $R_5$ Wasserstoff oder einen Kohlenwasserstoffrest darstellt, ausgewählt aus Alkylresten im Sinne des Chemielexikons von Duval (Dictionnaire de Chimie Duval) und Arylresten, vorzugsweise mit 1 bis 2 Kohlenstoffatomen;
- Z Wasserstoff oder einen Acylrest, vorzugsweise mit höchstens 10 Kohlenstoffatomen, darstellt.

**16.** Verfahren nach Anspruch 14 oder 15, dadurch gekennzeichnet, daß die Verbindung der Formel (V) ausgewählt ist aus desaktivierten Anilinen der folgenden Formel (II)

$$A\text{-}Ar\text{-}N\text{-}H_2 \quad \text{(II)}$$

wobei:

- A einen Substituenten, vorzugsweise eine ziehende Gruppe oder ein Wasserstoffatom, darstellt;
- Ar dieselbe Bedeutung, wie zuvor angegeben, hat.

**17.** Verfahren nach Anspruch 14 oder 15, dadurch gekennzeichnet, daß die Verbindung der Formel (V) ausgewählt ist aus Phenolen mit der folgenden Formel (IV)

$$(R)_n Ar\text{-}O\text{-}H \quad \text{(IV)}$$

wobei:

- Ar, R und n dieselbe Bedeutung, wie zuvor angegeben, haben.

**18.** Verfahren nach einem der Ansprüche 14 bis 17, dadurch gekennzeichnet, daß das Koordinierungsreagenz ein Pnictin ist, vorzugsweise ausgewählt ist aus mittels Pfropfung wasserlöslich gemachten Trialkylphosphinen und Triphenylphosphinen.

**19.** Verfahren nach einem der Ansprüche 14 bis 18, dadurch gekennzeichnet, daß man das Nucleophil der Formel (V) einem Reagenz gemäß einem der Ansprüche 1 bis 13 unterwirft.

**20.** Verfahren nach einem der Ansprüche 17 bis 19, dadurch gekennzeichnet, daß, wenn das Nucleophil der Formel (V) mindestens ein Halogen aufweist, das Element aus der Gruppe VIII Platin ist.

**21.** Verfahren nach Anspruch 19 oder 20, dadurch gekennzeichnet, daß das Phosphin und das Metall aus der Gruppe VIII in Form eines Tetrakisphosphinmetalls vorliegen.

**22.** Verfahren nach einem der Ansprüche 14 bis 21, dadurch gekennzeichnet, daß das Element aus der Gruppe VIII ein Metall aus der Platinmine ist, vorzugsweise Palladium.

**23.** Verfahren nach einem der Ansprüche 14 bis 22, dadurch gekennzeichnet, daß der Katalysator außerdem ein Oxid eines Elements aus der Gruppe V B des Periodensystems Elemente enthält.

**24.** Verfahren nach Anspruch 23, dadurch gekennzeichnet, daß das Element aus der Gruppe V B im dreiwertigen Zustand vorliegt.

**25.** Verfahren nach Anspruch 21 oder 22, dadurch gekennzeichnet, daß das Element aus der Gruppe V B Arsen ist.

**26.** Verfahren nach einem der Ansprüche 14 bis 25, dadurch gekennzeichnet, daß das Koordinierungsreagenz ein wasserlösliches Phosphin ist, vorzugsweise ein Triphenylphosphintrisulfonat, insbesondere des Natriums.

**27.** Verfahren nach einem der Ansprüche 14 bis 26, dadurch gekennzeichnet, daß die Reaktion in einem Milieu stattfindet, das zwei nichtmischbare flüssige Phasen umfaßt, von denen eine Phase eine wäßrige Phase ist, die das

Koordinierungsreagenz enthält.

**28.** Verfahren nach Anspruch 27, dadurch gekennzeichnet, daß die wäßrige Phase nach der Allylierungsreaktion wiedergewonnen wird und für eine erneute Allylierungsreaktion wiederverwendet wird.

## Claims

**1.** Allylation reagent containing:

- an aqueous phase;
- an allyl alcohol or an allyl derivative;
- a catalyst containing at least one element of group VIII of the Periodic Classification of the elements, the said element of group VIII of the Periodic Classification being maintained in the aqueous phase by complexation with at least one water-soluble coordinating agent (or ligand); with the condition that, when the coordinating agent is used in combination with a nitrile, the said allyl derivative is neither an ester of carbonic acid nor an ester of acetic acid.

**2.** Reagent according to claim 1, characterized in that the said reagent additionally contains a solvent A which is completely immiscible with the said aqueous liquid phase.

**3.** Reagent according to one of claims 1 and 2, characterized in that it additionally contains a solvent providing minimum solubility of the lipophilic substrates.

**4.** Reagent according to one of claims 1 to 3, characterized in that the said allyl alcohol or an allyl derivative is chosen from the derivatives of following formula (III):

$$Z\text{-}O\text{-}C(R_1)(R_2)\text{-}C(R_3){=\!=}(R_4)(R_5) \qquad (III)$$

where:

- $R_1$ represents a hydrogen or a hydrocarbon radical chosen from alkyl radicals within the meaning of the Duval dictionary of chemistry and aryl radicals;
- $R_2$ represents a hydrogen or a hydrocarbon radical chosen from alkyl radicals within the meaning of the Duval dictionary of chemistry and aryl radicals;
- $R_3$ represents a hydrogen or a hydrocarbon radical chosen from alkyl radicals within the meaning of the Duval dictionary of chemistry and aryl radicals;
- $R_4$ represents a hydrogen or a hydrocarbon radical chosen from alkyl radicals within the meaning of the Duval dictionary of chemistry and aryl radicals;
- $R_5$ represents a hydrogen or a hydrocarbon radical chosen from alkyl radicals within the meaning of the Duval dictionary of chemistry and aryl radicals;

and Z represents a hydrogen or a residue derived from an alcohol by removal of a hydroxyl group;
the total carbon number of the allyl derivative being at most equal to 50, advantageously to 30, carbon atoms.

**5.** Reagent according to one of claims 1 to 4, characterized in that the said coordinating agent is a pnictine, advantageously chosen from trialkylphosphines and triphenylphosphines rendered water-soluble by grafting, preferably triphenylphosphinetrisulphonates.

**6.** Reagent according to one of claims 1 to 5, characterized in that, when the molecule to be allylated exhibits at least one halogen, the said element of group VIII is platinum.

**7.** Reagent according to one of claims 5 and 6, characterized in that the said phosphine and the said metal of group VIII are in the tetrakisphosphinemetal form.

**8.** Reagent according to one of claims 1 to 7, characterized in that the element of group VIII is a platinum ore metal, advantageously palladium.

**9.** Reagent according to one of claims 1 to 8, characterized in that the said catalyst additionally contains an oxide of an element of group Vb of the Periodic Classification of the elements.

**10.** Reagent according to claim 9, characterized in that the said element of group Vb is in the trivalent state.

**11.** Reagent according to one of claims 9 and 10, characterized in that the said element of group Vb is arsenic.

**12.** Reagent according to one of claims 4 to 11, characterized in that at least 2, advantageously 3, of the $R_1$ to $R_5$ radicals have at most two carbon atoms.

**13.** Reagent according to claims 1 to 12, characterized in that it contains 2 immiscible liquid phases, one of which is the said aqueous phase containing the water-soluble phosphine.

**14.** Process for allylating a nucleophile, in particular phenols and anilines, characterized in that the said nucleophile is reacted with an allyl alcohol or an allyl derivative in the presence of a catalyst containing at least one element of group VIII of the Periodic Classification of the elements, the said element being maintained in the aqueous phase by complexation with at least one water-soluble coordinating agent (or ligand) and in that the said nucleophile is a compound of following formula (V):

$$(R)_n\text{Ar-Y-H} \qquad \text{(V)}$$

in which:

- Ar represents a homo- or heterocyclic, condensed or non-condensed, mono- or polycyclic aromatic radical;
- R represents at least one substituent chosen independently from chlorine, bromine, saturated or unsaturated linear, branched or cyclic alkyl radicals which are optionally substituted, or ether, optionally substituted alkoxy, optionally substituted aryl, aryloxy, amino, hydroxyl, carboxylate, acyloxy, ester, amido, nitrile or acid radicals;
- n is equal to 0 or to an integer equal to 1, 2, 3 or 4,
- Y represents a chalcogen atom, preferably of oxygen, or an NR' group with R' representing an acyl or an alkyl which is light in nature (from 1 to 10, preferably from 1 to 6, carbon atoms) or an aryl radical or advantageously a hydrogen atom,

with the condition that, when Y is NR', the compound has an anilide functional group or an aniline functional group deactivated by the presence of an electron-withdrawing group and/or by the presence of a heteroatom in the nucleus.

**15.** Process according to claim 14, characterized in that the said allyl alcohol or an allyl derivative is chosen from the derivatives of following formula (III):

$$\text{Z-O-C}(R_1)\,(R_2)\,\text{-C}(R_3)\text{==C}(R_4)\,(R_5) \qquad \text{(III)}$$

where:

- $R_1$ represents a hydrogen or a hydrocarbon radical chosen from alkyl radicals within the meaning of the Duval dictionary of chemistry and aryl radicals, preferably containing 1 or 2 carbon atoms;
- $R_2$ represents a hydrogen or a hydrocarbon radical chosen from alkyl radicals within the meaning of the Duval dictionary of chemistry and aryl radicals, preferably containing 1 or 2 carbon atoms;
- $R_3$ represents a hydrogen or a hydrocarbon radical chosen from alkyl radicals within the meaning of the Duval dictionary of chemistry and aryl radicals, preferably containing 1 or 2 carbon atoms;
- $R_4$ represents a hydrogen or a hydrocarbon radical chosen from alkyl radicals within the meaning of the Duval dictionary of chemistry and aryl radicals, preferably containing 1 or 2 carbon atoms;
- $R_5$ represents a hydrogen or a hydrocarbon radical chosen from alkyl radicals within the meaning of the Duval dictionary of chemistry and aryl radicals, preferably containing 1 or 2 carbon atoms;
- Z represents a hydrogen or an acyl radical, preferably of at most 10 carbon atoms.

**16.** Process according to one of claims 14 and 15, characterized in that the said compound of formula (V) is chosen from deactivated anilines of following formula (II):

$$A\text{-}Ar\text{-}N\text{-}H_2 \qquad \text{(II)}$$

where:

- A represents a substituent, preferably a withdrawing group, or a hydrogen atom;
- Ar has the same meaning as above.

**17.** Process according to claims 14 and 15, characterized in that the said compound of formula (V) is chosen from phenols exhibiting the following formula (IV):

$$(R)_n Ar\text{-}O\text{-}H \qquad \text{(IV)}$$

where:

- Ar, R and n have the same meaning as above.

**18.** Process according to one of claims 14 to 17, characterized in that the said coordinating agent is a pnictine, advantageously chosen from trialkylphosphines and triphenylphosphines rendered water-soluble by grafting.

**19.** Process according to claims 14 to 18, characterized in that the nucleophile of formula (V) is subjected to a reagent according to one of claims 1 to 13.

**20.** Process according to one of claims 17 to 19, characterized in that, when the nucleophile of formula (V) has at least one halogen, the said element of group VIII is platinum.

**21.** Process according to one of claims 19 and 20, characterized in that the said phosphine and the said metal of group VIII are in the tetrakisphosphinemetal form.

**22.** Process according to one of claims 14 to 21, characterized in that the element of group VIII is a platinum ore metal, advantageously palladium.

**23.** Process according to one of claims 14 to 22, characterized in that the said catalyst additionally contains an oxide of an element of group Vb of the Periodic Classification of the elements.

**24.** Process according to claim 23, characterized in that the said element of group Vb is in the trivalent state.

**25.** Process according to one of claims 21 and 22, characterized in that the said element of group Vb is arsenic.

**26.** Process according to one of claims 14 to 25, characterized in that the said coordinating agent is a water-soluble phosphine, preferably a triphenylphosphine-trisulphonate, advantageously sodium triphenylphosphine-trisulphonate.

**27.** Process according to one of claims 14 to 26, characterized in that the reaction takes place in a medium containing 2 immiscible liquid phases, one of which is an aqueous phase containing the said coordinating agent.

**28.** Process according to claim 27, characterized in that, after the allylation reaction, the aqueous phase is recovered and reused in a new allylation operation.

FIGURE UNIQUE